**Europäisches Patentamt**

**European Patent Office**

**Office européen des brevets**

(19)

(11) Veröffentlichungsnummer: **0 103 073 B1**

(12) **EUROPÄISCHE PATENTSCHRIFT**

(45) Veröffentlichungstag der Patentschrift:
**29.01.86**

(51) Int. Cl.⁴: **F 04 B 43/08, A 61 M 1/00**

(21) Anmeldenummer: **83105573.6**

(22) Anmeldetag: **07.06.83**

(54) **Schlauchpumpe, insbesondere für medizinische Anwendungen.**

(30) Priorität: **20.07.82 DE 3227051**

(43) Veröffentlichungstag der Anmeldung:
**21.03.84 Patentblatt 84/12**

(45) Bekanntmachung des Hinweises auf die Patenterteilung:
**29.01.86 Patentblatt 86/5**

(84) Benannte Vertragsstaaten:
**AT BE CH DE FR GB IT LI LU NL SE**

(56) Entgegenhaltungen:
**CH - A - 255 537**
**DE - B - 2 141 352**
**FR - A - 2 079 955**
**US - A - 2 915 983**
**US - A - 3 720 489**

(73) Patentinhaber: **INTERMEDICAT GMBH,**
**Gerliswilstrasse 74, CH-6020 Emmenbrücke (CH)**

(72) Erfinder: **Schubert, Ernst W., Dorotheenstrasse 6,**
**D-2400 Lübeck (DE)**
Erfinder: **Dörfler, Josef, Talblick 2,**
**D-3508 Melsungen 5 (DE)**

(74) Vertreter: **von Kreisler, Alek, Dipl.-Chem. et al,**
**Deichmannhaus am Hauptbahnhof, D-5000 Köln 1 (DE)**

## Beschreibung

Die Erfindung betrifft eine Schlauchpumpe, insbesondere für medizinische Anwendungen, mit einem Gehäuse, in dem eine Antriebswelle gelagert ist, einem von der Antriebswelle schräg abstehenden Wellenstück, einer auf dem Wellenstück gelagerten und gegenüber dem Gehäuse im wesentlichen verdrehungssicher festgehaltenen Taumelscheibe, einem an der Stirnseite der Taumelscheibe anliegenden ringförmigen Schlauch, dessen Enden einen Einlass und einen Auslass bilden, und mit einem an dem Gehäuse angebrachten, den Schlauch abstützenden Deckel.

Die Zufuhr von Flüssigkeitsmengen in den Körper von Patienten erfolgt in vielen Fällen aus einem Vorratsbehälter, aus dem die Flüssigkeit durch Schwerkraftwirkung ausläuft. Wenn höhere Genauigkeiten erforderlich sind, oder der Förderdruck durch die Schwerkraft nicht ausreicht, werden Verdrängerpumpen eingesetzt. Die mit den Flüssigkeiten in Kontakt kommenden Bauteile solcher Verdrängerpumpen werden dabei in der Regel als Kunststoffartikel zur einmaligen Verwendung ausgeführt, die anschliessend an die Benutzung fortgeworfen werden. Diese Kunststoffartikel sollten möglichst einfach herstellbar und billig zu fertigen sein, jedoch die medizinisch notwendige Genauigkeit und Sicherheit garantieren.

Bekannt ist eine Schlauchpumpe (US-A Nr. 2915983), bei der in einem Gehäuse eine Antriebswelle gelagert ist, die an ihrem Ende ein exzentrisch angeordnetes, schräg abstehendes Wellenstück aufweist. Auf dem Wellenstück ist eine Taumelscheibe gelagert, die relativ zu dem Gehäuse der Schlauchpumpe gegen Drehung gesichert ist. Zwischen der Taumelscheibe und einem das Gehäuse abschliessenden Deckel ist ein ringförmig verlegter Schlauchabschnitt angeordnet, dessen Enden durch Schlitze des Deckels hindurchgeführt sind. Die aus dem Deckel herausgeführten Schlauchenden werden mit einer Flüssigkeitsquelle und einem Verbraucher verbunden. Wenn sich die Antriebswelle dreht, wird die Taumelscheibe in eine Taumelbewegung versetzt, wobei sie einen Bereich des ringförmigen Schlauchabschnitts gegen den Deckel drückt und flachquetscht. Der flachgequetschte Bereich wandert während der Taumelbewegung, so dass die in dem ringförmigen Schlauchabschnitt enthaltene Flüssigkeit in dem Schlauch vorgetrieben wird.

Die bekannten Schlauchpumpen haben den Nachteil, dass das Einlegen des Schlauches schwierig ist und eine gewisse Übung erfordert. Ein Abschnitt des Schlauches muss in dem Deckel bzw. über der Taumelscheibe ringförmig verlegt werden, während die sehr langen Enden aus Öffnungen des Deckels herausgeführt werden. Dabei ist der Durchmesser des ringförmigen Schlauchabschnitts nicht fest vorgegeben, so dass die Lage des ringförmigen Schlauchabschnitts in der Schlauchpumpe undefiniert ist. Wenn der Durchmesser dieses ringförmigen Schlauchabschnitts zu gross oder zu klein gewählt wird, treten bei der umlaufenden Abquetschung durch die Taumelscheibe Relativbewegungen zwischen dem ringförmigen Schlauchstück einerseits und dem Deckel oder der Taumelscheibe andererseits auf. Diese Relativbewegungen führen zu einer erhöhten Reibung und zu Abnutzung und Leistungsverlusten. Schliesslich erfordert bei den bekannten Schlauchpumpen das Einlegen des Schlauches eine gewisse Erfahrung. Wenn der Schlauch mit falschem Richtungssinn eingelegt wird, fördert die Pumpe in die falsche Richtung.

Bekannt ist ferner eine Schlauchpumpe (US-A Nr. 3720489), bei der eine Taumelscheibe den Deckel des Gehäuses bildet. Der ringförmige Schlauchabschnitt wird in einer Ringnut des Gehäuses verlegt. Die Taumelscheibe wird von einer Feder in Richtung auf die Ringnut gedrückt. Auch bei dieser Schlauchpumpe wird der Schlauch in beliebiger Weise eingelegt, wobei die aus dem Gehäuse herausragenden langen Schlauchenden in beliebiger Weise angeschlossen werden können. Auch diese Schlauchpumpe bietet nicht die Möglichkeit des einfachen Auswechselns des Schlauches und stellt nicht sicher, dass der Schlauch nur in einer definierten Lage eingelegt werden kann, um eine unbeabsichtigte Umkehr der Förderrichtung zu vermeiden.

Bei medizinischen Anwendungen, bei denen die Schlauchpumpe beispielsweise Blut oder ein Serum fördern soll, wird der Schlauch als Einmalartikel benutzt. Der Schlauch muss sich daher aus der Schlauchpumpe einfach entfernen und ersetzen lassen. Da ein fehlerhaftes Einlegen des Schlauches bei derartigen Anwendungen sehr schwerwiegende Folgen für den Patienten haben kann, muss sichergestellt sein, dass ein Betrieb der Schlauchpumpe nur bei richtig eingelegtem Schlauch möglich ist.

Der Erfindung liegt die Aufgabe zugrunde, eine Schlauchpumpe der eingangs genannten Art zu schaffen, bei der der Schlauch auf einfache Weise ersetzt werden kann und deren Handhabung auch durch technische Laien, wie z.B. das Pflegepersonal eines Krankenhauses oder den Patienten, problemlos durchgeführt werden kann.

Zur Lösung dieser Aufgabe ist erfindungsgemäss vorgesehen, dass der Deckel an seiner Unterseite eine ringförmige Nut zum Festlegen des ringförmigen Schlauchabschnitts sowie eine Klemmeinrichtung zum Fixieren des Schlauchs im Deckel aufweist, dass die Schlauchenden mit einem in den Deckel einsetzbaren gemeinsamen Anschlussstück verbunden sind, das Anschlüsse für Zulauf und Ablauf aufweist, dass der Deckel auf der dem Anschlussstück gegenüberliegenden Seite über eine Gelenkachse mit dem Gehäuse verbunden ist und dass in der Nähe des Anschlussstückes an dem Gehäuse eine Verriegelungsvorrichtung zum Verriegeln des Deckels in der Verschlussstellung angeordnet ist.

Bei der erfindungsgemässen Schlauchpumpe sind beide Enden des Schlauches mit einem gemeinsamen Anschlussstück verbunden, das zusammen mit dem Schlauch in eine Ausnehmung des Deckels eingesetzt wird. Der Schlauch bildet also zusammen mit dem Anschlussstück eine Wegwerfeinheit. Das Anschlussstück stellt sicher, dass der Schlauch in der richtigen Lage und Förderrichtung in den Deckel eingesetzt wird. Das Auswechseln des Schlauches erfolgt, nachdem der Deckel um die Gelenkachse herum hochgeklappt ist. Zum Auswechseln des Schlauches sind lediglich einfache Handgriffe erforderlich. Die Verriegelungsvorrichtung wird gelöst und der Deckel hochgeklappt. Dann wird der Schlauch zusammen mit dem Anschlussstück aus dem Deckel entnommen und durch einen neuen Schlauch mit Anschlussstück ersetzt. Die ringförmige Nut bewirkt die exakte Positionierung des ringförmigen Schlauchabschnitts im Deckel, so dass keine wesentlichen Verschiebungen des Schlauches beim Betrieb auftreten und insbesondere Reibungsbeanspruchungen in Umfangsrichtung des Schlauches vermieden werden. Durch die klappbare Anordnung des Deckels am Gehäuse wird das Einlegen des Schlauches erleichtert. Der Deckel ist unverlierbar angeordnet und während der Schliessbewegung exakt geführt. Hierdurch wird vermieden, dass der Schlauch sich beim Schliessen des Deckels verschieben und sich dadurch verklemmen kann.

Bei Schlauchpumpen besteht die Gefahr, dass die Förderung kurzzeitig aussetzt, so dass sogar ein Rückzug der Flüssigkeit eintritt, wenn das Quetschelement das auslassseitige Ende des Schlauches überstreicht und dieses Ende sich anschliessend zum vollen Schlauchquerschnitt zurückbildet. Um die hierdurch hervorgerufenen Diskontinuitäten bei der Förderung zu vermeiden oder mindestens abzuschwächen, können zwei Schläuche seitlich nebeneinander in einer Ebene angeordnet sein, wobei jedem Schlauch eine eigene Taumelscheibe zugeordnet ist. Die Taumelscheiben sind von einem gemeinsamen Antrieb mit gleichen Geschwindigkeiten angetrieben und beide Schläuche weisen ein gemeinsames Anschlussstück mit einem einzigen Zulauf und einem einzigen Ablauf auf. Auf diese Weise werden zwei Schlauchpumpen derart miteinander kombiniert, dass ihre Schläuche zusammen mit dem Anschlussstück eine einzige Wegwerfeinheit bilden. Beide Schläuche können in einem gemeinsamen Deckel angeordnet werden. Die Taumelscheiben beider Schläuche sind phasenmässig versetzt oder gegenphasig angetrieben, so dass die kritischen Phasen der kurzzeitigen Unterbrechung der Förderung nicht zusammenfallen.

Im folgenden werden unter Bezugnahme auf die Zeichnungen Ausführungsbeispiele der Erfindung näher erläutert.

Es zeigen:

Fig. 1 einen Längsschnitt durch eine Schlauchpumpe,

Fig. 2 einen Schnitt entlang der Linie II-II, in Fig. 1,

Fig. 2a eine Darstellung wie Fig. 2 bei einer abgeänderten Ausführungsform,

Fig. 3 eine teilweise Stirnansicht der Schlauchpumpe aus Richtung des Pfeiles III in Fig. 1,

Fig. 4 einen Längsschnitt durch den Arbeitsteil einer Doppel-Schlauchpumpe, und

Fig. 5 eine Ansicht des Deckels der Doppel-Schlauchpumpe nach Fig. 4 in geöffnetem Zustand mit eingelegten Schläuchen.

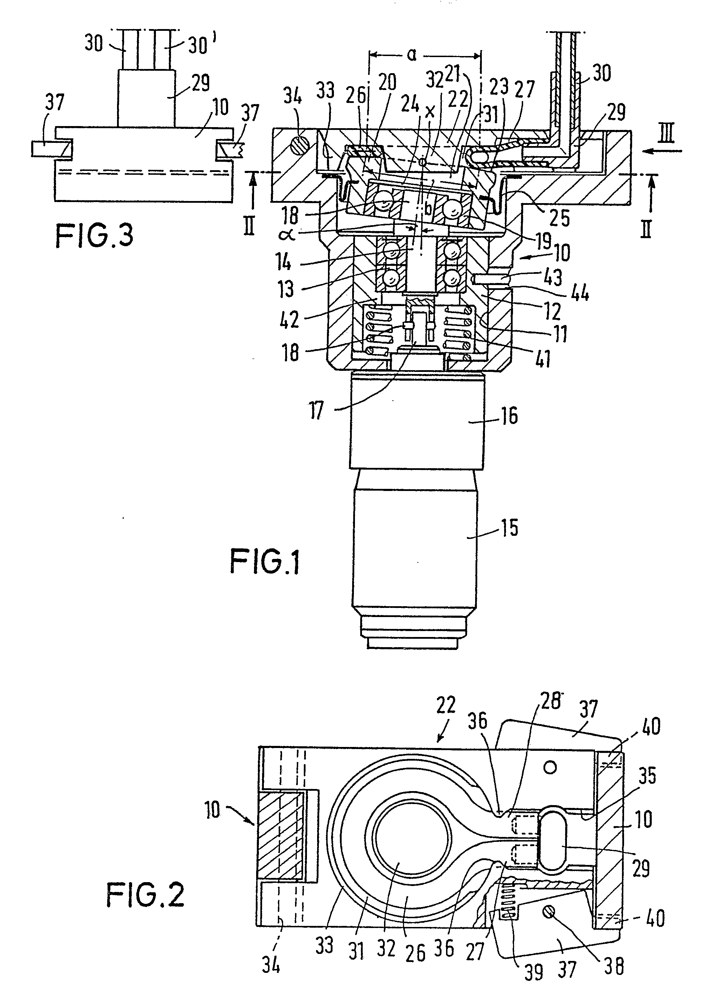

Die in den Fig. 1 bis 3 dargestellte Schlauchpumpe weist ein Gehäuse 10 mit einer zylindrischen Bohrung 11 auf, in der eine Hülse 12 längsverschiebbar gelagert ist. Im Innern der Hülse 12 ist mit Kugellagern 13 die Welle 14 gelagert, welche von einem Elektromotor 15 über ein Untersetzungsgetriebe 16 angetrieben ist. Die Antriebswelle 17 wird von der Ausgangswelle des Untersetzungsgetriebes 16 gebildet und sie ist über eine gabelförmige Kupplung 18 derart mit der Welle 14 gekuppelt, dass die Welle 14 kleine Axialverschiebungen relativ zur Ausgangswelle 17 ausführen kann.

An ihrem abtriebsseitigen Ende weist die Welle 14 ein abgewinkeltes Wellenstück 18 auf, dessen Achse mit der Achse der Welle 14 einen spitzen Winkel α bildet. Auf diesem Wellenstück 18 ist über ein Kugellager 19 die Taumelscheibe 20 gelagert. Das Kugellager 19 und die Taumelscheibe 20 befinden sich in einer Kammer 21 im Innern des Gehäuses 10. Diese Kammer kann mit dem Deckel 22 verschlossen werden. Die Taumelscheibe 20 besteht bei dem vorliegenden Ausführungsbeispiel aus einem Ring, dessen äussere Stirnseite 23 entsprechend dem Winkel α abgeschrägt ist. Der Schnittpunkt der Achsen von Welle 14 und Wellenstück 18 liegt in der Ebene des mittleren Durchmessers b der Stirnseite 23 (d.h. des Durchmessers des Mittelkreises der konischen Fläche der Stirnseite 23).

Im Innern des Ringes sitzt das Kugellager 19, dessen äussere Stirnseite durch eine Dichtscheibe 24 abgedichtet ist, so dass keine Flüssigkeit durch die Axialbohrung der Taumelscheibe 20 hindurchdringen kann. Am Umfang der Taumelscheibe 20 ist der innere Rand einer Membran 25 befestigt, deren äusserer Rand an der Seitenwand der Kammer 21 des Gehäuses 10 angebracht ist. Die Dichtungsscheibe 24 bildet daher zusammen mit der Membran eine flüssigkeitsdichte Abdichtung der Antriebseinrichtung der Taumelscheibe 20 gegen den den Schlauch 26 aufnehmenden Raum.

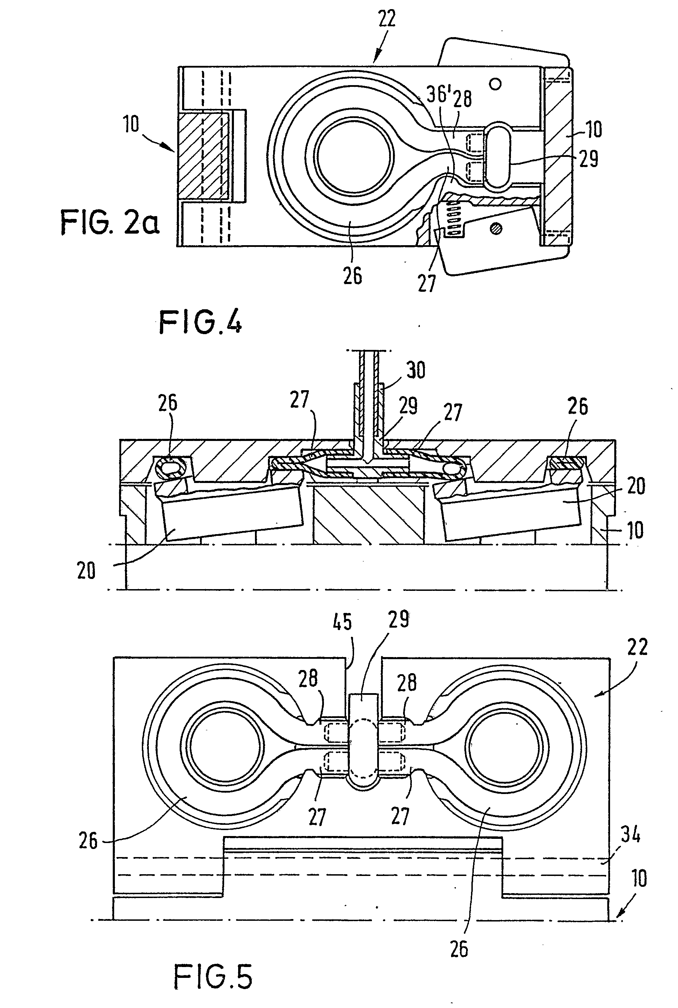

Der Schlauch 26 ist an der Unterseite des Deckels 22 befestigt. Wie aus Fig. 2 hervorgeht, ist der Schlauch 26 zu einem Ring gebogen, wobei der Einlass 27 und der Auslass 28 nebeneinander angeordnet sind und radial abstehen. Der Einlass 27 und der Auslass 28 sind mit einem Anschlussstück 29 verbunden, das im wesentlichen L-förmig ausgebildet ist und dessen Zulaufstutzen 30 und Ablaufstutzen 30' senkrecht aus dem Deckel 22 herausragen.

Der Schlauch 26 ist in eine ringförmige Nut 31 an der Unterseite des Deckels 22 eingelegt. Diese ringförmige Nut 31 wird innen durch einen axial vorstehenden Vorsprung 32 und aussen durch einen umlaufenden Rand 33 von gleicher Tiefe begrenzt. Wie aus Fig. 1 ersichtlich ist, greift die ringförmige Taumelscheibe 20 in die ringförmige Nut 31 des Deckels 22 ein, wobei infolge der Schrägstellung der Taumelscheibe 20 die Eindringtiefe über den Umfang variiert. Zwischen der äusseren Stirnseite 23 der Taumelscheibe und dem Boden der ringförmigen Nut 31 wird der Schlauch 26 in axialer Richtung zusammengequetscht.

Wie aus Fig. 1 hervorgeht, ist der mittlere Umfang (Durchmesser a) der ringförmigen Stirnseite 23 der Taumelscheibe 20 gleich gross wie der mittlere Umfang (Durchmesser b) der ringförmigen Nut 31 bzw. der mittlere Umfang des Schlauchringes. Dadurch wird erreicht, dass die Stirnseite 23 eine reine Rollbewegung in der Nut 31 ausführt, ohne sich jedoch in dieser Nut zu drehen. Würden die mittleren Durchmesser a und b stärker voneinander abweichen, so würde die Taumelscheibe 20 dazu neigen, sich in dem Gehäuse langsam um die Achse des Wellenstückes 18 herum zu drehen. Dabei würde sie eine Schubwirkung auf den Schlauch ausüben, wodurch Reibungsverluste entstehen würden, die den Wirkungsgrad verschlechtern.

Der Deckel 22 ist an dem Gehäuse 10 mit einer Gelenkachse 34 befestigt, so dass er gemäss Fig. 2 aufgeklappt werden kann. Das Anschlussstück 29 befindet sich an dem der Gelenkachse 34 abgewandten Ende des Deckels 22. An diesem Ende ist ein Längsschlitz 35 vorgesehen. Der Schlauch 26, der von unten her in den Deckel 22 eingelegt ist, wird durch Laschen 36 festgehalten, die das einlassseitige Ende und das auslassseitige Ende des Schlauches 26 teilweise übergreifen. Zwischen diesen Laschen 36 befindet sich jedoch ein Spalt, der so breit ist, dass der Schlauch 26 unter relativ geringer Verformung aus dem Deckel 22 herausgenommen werden kann.

Die Ausführungsform der Fig. 2a entspricht weitgehend derjenigen der Fig. 2. Der einzige Unterschied besteht darin, dass anstelle der Laschen 36, die gemäss Fig. 2 den Schlauch 26 in dem Deckel festhalten, am Rand des Längsschlitzes 35 des Deckels 12 eine halbzylindrische Beule 36' vorgesehen ist, die das eine Schlauchende in der Nähe des Anschlussstückes 29 in Richtung auf das andere Schlauchende drückt. Auf diese Weise wird der Schlauch deformiert, wobei beide Schlauchenden leicht zusammengedrückt und in dem Deckel 22 durch Klemmung festgehalten werden.

Zur Verriegelung des Deckels 22 in der Schliessstellung am Gehäuse 10 dienen Klauen 37 an dem der Gelenkachse 34 abgewandten Ende des Deckels 22. Diese Klauen 37 sind jeweils mit einem Gelenkzapfen 38 an dem Deckel 22 gelagert und werden von einer Feder 39 in die Verriegelungsstellung gedrückt. Ihre äusseren Enden 40 greifen gemäss Fig. 3 in seitliche Ausnehmungen des Gehäuses 10 ein, um den Deckel 22 in der Schliessstellung an dem Gehäuse 10 zu verriegeln. Werden die Enden 40 durch seitliches Eindrücken der Klauen 37 unter Zusammendrückung der Federn 39 auseinandergespreizt, dann wird der Deckel 22 am Gehäuse 10 entriegelt und er kann hochgeschwenkt werden.

Zur Ingebrauchnahme der Schlauchpumpe wird zunächst der Schlauch 26 zusammen mit dem Anschlussstück 29 in der in Fig. 2 dargestellten Weise in den Deckel 22 eingelegt. Dann wird der Deckel 22 geschlossen und an dem Gehäuse 10 verriegelt.

Hierbei wird die Taumelscheibe 20 in ihrem am weitesten vorstehenden Bereich so fest gegen den Schlauch 26 gedrückt, dass sie diesen in der ringförmigen Nut 31 zusammenquetscht. Auf diese Weise entsteht entlang des Umfangs des von dem Schlauch 26 gebildeten Ringes eine einzige Quetschstelle. Diese ist in Umfangsrichtung so lang, dass an Ein- und Auslass kein Kurzschluss entsteht; es werden kurzzeitig beide Schlauchenden 27 und 28 gemeinsam abgequetscht. Wird der Motor 15 eingeschaltet, dann dreht sich die Welle 14 und die Taumelscheibe 20 führt eine Taumelbewegung aus, ohne jedoch an der Drehung teilzunehmen. Infolge der Taumelbewegung wandert die Stelle, an der die maximale Zusammendrückung des Schlauches 26 stattfindet, entlang des von dem Schlauch 26 gebildeten Ringes mit konstanter Geschwindigkeit um. Auf diese Weise wird die im Schlauch 26 befindliche Flüssigkeit vom Einlass 27 zum Auslass 28 vorgetrieben.

Nach Beendigung der Behandlung werden die Klauen 37 gelöst und der Deckel 22 wird hochgeklappt. In diesem Zustand kann der Schlauch 26 zusammen mit dem Anschlussstück 29 entfernt und ersetzt werden.

Die Kugellager 13 und 24 zur Lagerung der Welle 14 bzw. der Taumelscheibe 20 dienen nicht nur als Radialkugellager, sondern sie sind auch imstande, axiale Kräfte zu übertragen, damit der Anpressdruck der Feder 41 auf die Taumelscheibe 20 übertragen wird. Diese Feder 41 stützt sich an der rückwärtigen Stirnwand des Gehäuses 10 ab und drückt gegen eine Ringschulter 42 der Hülse 12, so dass die Hülse 12 zusammen mit der Welle 14 und den Kugellagern 13 und 19 sowie mit der Taumelscheibe 20 in Richtung auf den Schlauch 26 bzw. den Deckel 22 gedrückt wird. Um die Hülse 12 verdrehungssicher in dem Gehäuse 10 festzulegen, ist an der Hülse 12 ein radial abstehender Stift 43 vorgesehen, der in ein axiales Langloch 44 in der Wand des Gehäuses 10 hineinragt. Dieser Stift 43 dient auch als axialer Anschlag, damit die Taumelscheibe bei geöffnetem Deckel nicht herausfällt und damit die Taumelscheibe bei geschlossenem Deckel, ohne Schlauch, nicht mit den Führungen 32 und 33 in Beziehung kommt und Beschädigungen hervorruft.

Da sich derjenige Teil der Taumelscheibe 20, der axial am weitesten vorsteht und die maximale Abquetschung des Schlauches 26 bewirkt, auf dem von dem Schlauch gebildeten Kreis umläuft, wird die Flüssigkeit von dem Einlass 27 zum Auslass 28 gefördert.

Bei dem Ausführungsbeispiel der Fig. 4 und 5 sind zwei Schlauchpumpen in einem gemeinsamen Gehäuse 10 nebeneinander angeordnet. Die Taumelscheiben 20 der beiden Schlauchpumpen sind gegensinnig zueinander angetrieben und ihre Einlässe 27 sind untereinander verbunden, ebenso wie ihre Auslässe 28 untereinander verbunden sind. Die Einlässe 27 sind mit einem Einlauf 30 des Anschlussstückes 29 verbunden, während die Auslässe 28 mit einem (nicht dargestellten) Ablauf des Anschlussstückes 29 verbunden sind. Das Anschlussstück 29 ist in einem Spalt 45 des Deckels 22 angeordnet, der an der der Gelenkachse 34 abgewandten Seite des Deckels 22 frei ausläuft.

Wie aus Fig. 4 hervorgeht, haben die beiden Taumelscheiben 20 eine gegenseitige Phasenverschiebung von 180°. Während die linke Taumelscheibe 20 die stärkste Abquetschung des zugehörigen Schlauches 26 gerade an der dem Anschlussstück 29 zugewandten Seite ausführt, bewirkt die rechte Taumelscheibe 20 die maximale Abquetschung zu derselben Zeit an derjenigen Stelle des zugehörigen Schlauches 26, die den grössten Abstand von dem Anschlussstück 29 hat.

Der Deckel 22 nimmt bei dem Ausführungsbeispiel der Fig. 4 und 5 beide Schläuche 26, die in einer gemeinsamen Ebene liegen, gemeinsam auf. Die Schläuche 26 bilden zusammen mit dem zwischen ihnen angeordneten Anschlussstück 29 die Wegwerfeinheit. Durch den phasenverschobenen Antrieb der beiden Taumelscheiben 20 werden etwaige zeitliche Diskontinuitäten der Gesamt-Fördermenge der Schlauchpumpe gemildert.

## Patentansprüche

1. Schlauchpumpe, insbesondere für medizinische Anwendungen, mit einem Gehäuse (10), in dem eine Antriebswelle (14) gelagert ist, einem von der Antriebswelle schräg abstehenden Wellenstück (18), einer auf dem Wellenstück (18) gelagerten und gegenüber dem Gehäuse (10) im wesentlichen verdrehungssicher festgehaltenen Taumelscheibe (20), einem an der Stirnseite der Taumelscheibe anliegenden ringförmigen Schlauch (26), dessen Enden einen Einlass und einen Auslass bilden, und mit einem an dem Gehäuse angebrachten, den Schlauch abstützenden Deckel (22), dadurch gekennzeichnet, dass der Deckel (22) an seiner Unterseite eine ringförmige Nut (31) zum Festlegen des ringförmigen Schlauchabschnitts und eine Klemmeinrichtung (36; 36') zum Fixieren des Schlauches im Deckel (22) aufweist, dass die Schlauchenden (27, 28) mit einem in den Deckel (22) einsetzbaren gemeinsamen Anschlussstück (29) verbunden sind, das Anschlüsse (30) für Zulauf und Ablauf aufweist, dass der Deckel auf der dem Anschlussstück (29) gegenüberliegenden Seite über eine Gelenkachse (34) mit dem Gehäuse (10) verbunden ist und dass in der Nähe des Anschlussstückes (29) an dem Gehäuse (10) eine Verriegelungsvorrichtung (37, 38, 39) zum Verriegeln des Deckels in der Verschlussstellung angeordnet ist.

2. Schlauchpumpe nach Anspruch 1, dadurch gekennzeichnet, dass das Anschlussstück (29) winkelförmig ausgebildet ist und mit einem Schenkel unter den Deckel (22) ragt, während der andere Schenkel aus einem Schlitz (35) des Deckels herausragt.

3. Schlauchpumpe nach Anspruch 1 oder 2, dadurch gekennzeichnet, dass die Breite der ringförmigen Nut (31) etwa derjenigen des zusammengequetschten Schlauchs (26) entspricht und dass die Taumelscheibe (20) aus einem axial in die Nut (31) eintauchenden Ring besteht.

4. Schlauchpumpe nach Anspruch 3, dadurch gekennzeichnet, dass der mittlere Durchmesser (a) der ringförmigen Nut (31) annähernd gleich gross ist wie der mittlere Durchmesser der gegen den Schlauch (26) drückenden Stirnseite (23) der Taumelscheibe (20).

5. Schlauchpumpe nach Anspruch 3, dadurch gekennzeichnet, dass die Achsen der Antriebswelle (14) und des Wellenstücks (18) sich in einem Punkt (x) schneiden, der annähernd in der Ebene des mittleren Durchmessers (b) der den Schlauch abstützenden ringförmigen Stirnseite (23) des Ringes liegt.

6. Schlauchpumpe nach einem der Ansprüche 1 bis 5, dadurch gekennzeichnet, dass die Umfangsfläche der Taumelscheibe (20) über eine Membran (25) abdichtend mit der Seitenwand eines Raumes des Gehäuses (10) verbunden ist, in dem die Taumelscheibe (20) untergebracht ist.

7. Schlauchpumpe, insbesondere für medizinische Anwendungen, mit einem Gehäuse (10), in dem eine Antriebswelle (14) gelagert ist, einem von der Antriebswelle schräg abstehenden Wellenstück (18), einer auf dem Wellenstück (18) gelagerten und gegenüber dem Gehäuse (10) im wesentlichen verdrehungssicher festgehaltenen Taumelscheibe (20), einem an der Stirnseite der Taumelscheibe anliegenden ringförmigen Schlauch (26), dessen Enden einen Einlass und einen Auslass bilden, und mit einem an dem Gehäuse angebrachten, den Schlauch abstützenden Deckel (22), dadurch gekennzeichnet, dass zwei Schläuche (26) seitlich nebeneinander in einer gemeinsamen Ebene angeordnet sind, dass jedem Schlauch eine eigene Taumelscheibe (20) zugeordnet ist, dass die Taumelscheiben (20) von einem gemeinsamen Antrieb mit gleichen Geschwindigkeiten angetrieben sind und dass beide Schläuche (26) ein gemeinsames Anschlussstück (29) mit einem einzigen Zulauf und einem einzigen Ablauf aufweisen.

8. Schlauchpumpe nach Anspruch 7, dadurch gekennzeichnet, dass beide Schläuche (26) in einem gemeinsamen Deckel (22) angeordnet sind.

9. Schlauchpumpe nach Anspruch 8, dadurch gekennzeichnet, dass die Taumelscheiben (20) beider Schläuche (26) phasenmässig versetzt oder gegenphasig angetrieben sind.

## Claims

1. Hose pump, in particular for medical purposes, comprising a housing (1) to support therein a driving shaft (14), a shaft piece (18) obliquely projecting from the driving shaft, a swash plate (20) supported on the shaft piece (18) and retained to be substantially non-rotatable relative to the housing (10), an annular hose (26) joined to the end side of the swash plate and whose ends form an inlet and an outlet, and a cover (22), that hose ends (27, 28) are conncted to a common connecting piece (29) insertable in the cover (22) mounted at the housing and supporting the hose, characterized in that the underside of the cover (22) contains an annular groove (31) for fixing the annular hose section, and a clamping means (36; 36') for fixing the hose in the cover (22), that the hose ends (27, 28) are connected to a common connecting piece (29) insertable in the cover (22) and having terminals (30) for inlet and for discharge, that the cover is connected to the housing (10) by a hinged axle (34) at the side opposite to the connecting piece (29), and that in the vicinity of the connecting piece (29), there is provided at the housing (10) a locking means (37, 38, 39) for locking the cover in the closure position.

2. Hose pump according to Claim 1, characterized in that the connecting piece (29) is angular, with one leg projecting beneath the cover (22) while the other leg extends out of a slot (35) of the cover.

3. Hose pump according to Claim 1 or 2, characterized in that the width of the annular groove (31) corresponds approximately to that of the squeezed hose (26), and that the swash plate (20) consists of a ring axially dipping into the groove (31).

4. Hose pump according to Claim 3, characterized in that the average diameter (a) of the annular groove (31) is nearly as large as the average diameter of the swash plate (20) end side (23) pressing against the hose (26).

5. Hose pump according to Claim 3, characterized in that the axles of the driving shaft (14) and of the shaft piece (18) intersect at a point (x) which is situated nearly in the plane of the average diameter (b) of the annular ring end side (23) supporting the hose.

6. Hose pump according to one of Claims 1 to 5, characterized in that the peripheral surface of the swash plate (20) is sealingly connected via a diaphragm (25) to the side wall of a chamber of the housing (10) in which the swash plate (20) is positioned.

7. Hose pump, in particular for medical purposes, comprising a housing (10) to support therein a driving shaft (14), a shaft piece (18) obliquely projecting from the driving shaft, a swash plate (20) supported on the shaft piece (18) and retained to be substantially non-rotatable relative to the housing (10), an annular hose (26) joined to the end side of the swash plate and whose ends form an inlet and an outlet, and a cover (22) mounted at the housing and supporting the cover (22), characterized in that two hoses (26) are mounted side by side in a common plane, that a swash plate (20) is assigned to each hose, that the swash plates (20) are driven at the same speeds by one common drive and that both hoses (26) have one common connecting piece (29) with one sole inlet and one sole outlet.

8. Hose pump according to Claim 7, characterized in that both hoses (26) are provided in one common cover (22).

9. Hose pump according to Claim 8, characterized in that the swash plates (20) of both hoses (16) are driven out-of-phase or in counterphase.

## Revendications

1. Pompe à tuyau flexible, en particulier pour des applications médicales, comportant un boîtier (10) dans lequel est logé un arbre d'entraînement (14), un embout (18) oblique par rapport à l'arbre d'entraînement, un plateau oscillant (20) monté pivotant sur l'embout (18) et sensiblement immobilisé en rotation par rapport au boîtier (10), un tuyau flexible (26) de forme annulaire disposé contre la face frontale du plateau oscillant (20) et dont les deux extrémités constituent respectivement une entrée d'admission et une sortie de refoulement, et un couvercle (22) de maintien du flexible (26) monté sur le boîtier, caractérisée en ce que:

le couvercle (22) comporte sur sa face inférieure une gorge annulaire (31) de maintien de la partie annulaire du tuyau, et un système de serrage (36; 36') pour la fixation du tuyau dans le couvercle (22), que les extrémités (27, 28) du tuyau sont reliées à un raccord commun (29) susceptible d'être inséré dans le couvercle (22) et comprenant des orifices de raccordement (30) pour l'aspiration et le refoulement, que le couvercle (22) est relié au boîtier (10) par un axe d'articulation (34) du côté opposé au raccord (29), et qu'un système de verrouillage (37, 38, 39) est disposé sur le boîtier (10) dans le voisinage du raccord (29), pour le verrouillage du couvercle (22) en position de fermeture.

2. Pompe à tuyau flexible selon la revendication 1, caractérisée en ce que le raccord (29) est coudé et comporte une branche engagée sous le couvercle (22), tandis que l'autre branche du raccord ressort par une fente (35) du couvercle (22).

3. Pompe à tuyau flexible selon l'une des revendications 1 ou 2, caractérisée en ce que la largeur de la gorge annulaire (31) du couvercle correspond sensiblement à celle de l'élément tubulaire (26) en état d'écrasement et que le plateau oscillant (20) se compose d'une bague pénétrant axialement, dans la gorge (31).

4. Pompe à tuyau flexible selon la revendication 3, caractérisée en ce que le diamètre moyen (a) de la gorge annulaire (31) est sensiblement égal au diamètre moyen de la face frontale (23) du plateau oscillant (20) s'appuyant contre le tuyau (26).

5. Pompe à tuyau flexible selon la revendication 3, caractérisée en ce que les axes de l'arbre d'entraînement (14) et de l'embout (18) se coupent en un point (x), qui est situé sensiblement dans le plan du diamètre moyen (b) de la face frontale annulaire (23) de la bague s'appuyant sur le tuyau (26).

6. Pompe à tuyau flexible selon l'une des revendications 1 à 5, caractérisée en ce que la face périphérique du plateau oscillant (20) est reliée de manière étanche par une membrane (25) à la paroi latérale d'un logement du boîtier (10) dans lequel est placé le plateau oscillant (20).

7. Pompe à tuyau flexible, en particulier pour des applications médicales, comportant un boîtier (10), dans lequel est logé un arbre d'entraînement (14), un embout (18) s'écartant en oblique de l'arbre d'entraînement, un plateau oscillant (20) monté rotatif sur l'embout (18) et sensiblement immobilisé en rotation par rapport au boîtier (10), un tuyau flexible (26) de forme annulaire, appliqué sur la face frontale du plateau oscillant et dont les extrémités constituent respectivement une entrée d'admission et une sortie de refoulement, et un couvercle (22) de maintien du tuyau, monté sur le boîtier (22), caractérisée en ce que deux tuyaux (26) sont disposés l'un à côté de l'autre dans un même plan, qu'un plateau oscillant particulier (20) est associé à chaque tuyau, que les plateaux oscillants (20) sont entraînés à des vitesses égales par un système d'entraînement commun et que les deux tuyaux (26) comportent un raccord commun (29) comprenant un seul orifice d'aspiration et un seul orifice de refoulement.

8. Pompe à tuyau flexible selon la revendication 7, caractérisée en ce que les deux tuyaux (26) sont disposés dans un couvercle commun (22).

9. Pompe à tuyau flexible selon la revendication 8, caractérisée en ce que les plateaux oscillants (20) des deux tuyaux (26) sont déphasés ou entraînés en opposition de phase.

FIG.3

FIG.1

FIG.2

0 103 073

FIG. 2a

FIG.4

FIG.5